# EUROPEAN PATENT SPECIFICATION

(11) **EP 0 614 675 B1**
(45) Date of publication and mention of the grant of the patent: **29.10.1997**
(21) Application number: 93120109.9
(22) Date of filing: 14.12.1993
(51) Int. Cl.: A61M 1/36, A61M 5/14, B29C 45/00

(54) **Drip and/or expansion chamber with intergral filter and method of manufacturing of such a chamber**
Tropfenkammer und/oder Expansionskammer mit integriertem Filter und Herstellungsverfahren von einer solchen
Chambre d'écoulement et/ou d'expansion à filtre intégré et procédé de fabrication de celle-ci

(30) Priority: 11.02.1993 SE 9300448
(43) Date of publication of application: 14.09.1994
(73) Proprietor: GAMBRO DIALYSATOREN GmbH & CO. KG, D-72373 Hechingen (DE)
(72) Inventor: Raabe, Herbert, D-72401 Haigerloch-Stetten (DE); Recktenwald, Gottfried, D-72793 Pfullingen (DE)
(74) Representative: Asketorp, Göran

(56) References cited:
- EP-A- 0 033 080
- DE-C- 3 641 644

## Description

### TECHNICAL FIELD:

The present invention relates to a drip chamber and/or expansion chamber comprising a housing with an upper part and a lower part, each of which being provided with one or more inlets and/or outlets, and at least one filter arranged between said parts, and a method for manufacturing such a chamber.

The chamber is preferably intended to be used for filtration of blood during extracorporeal blood treatment, for example during haemodialysis, haemodiafiltration or haemofiltration.

### BACKGROUND OF THE INVENTION:

Chambers for the above-stated purposes can be manufactured for example by blow moulding, as described in EP-B1-0 058 325. Such chambers are however difficult to provide with filters and are thus suitably combined with separate filters. A chamber according to the preamble portion of claim 1 is known from EP-A-0 033 080.

Alternatively, chambers can be manufactured by injection moulding. In such cases, the chambers have until now been provided with subsequently added filters which can be arranged in accordance with that described in the German document or can be arranged to be clamped between the chamber housing and an associated cap.

### SUMMARY OF THE INVENTION:

The present invention thus relates to a drip chamber and/or expansion chamber comprising a housing with an upper part and a lower part, each of which being provided with one or more inlets and/or outlets, and at least one filter arranged between said parts.

The chamber according to the invention is characterized in that at least one such filter is integrally formed with one of said parts. In this manner the number of components is reduced in comparison with conventional chambers provided with filters.

The filter is suitably manufactured simultaneously with its associated part by injection moulding in a single mould. Other methods of manufacture are however conceivable.

A simple and practical design is obtained when the housing consists of an elongated, preferably circular-cylindrical sleeve which, at both its ends, is closed by said parts, of which one or both can consist of a cap attached to the sleeve.

A simple design and simple moulding tools are obtained when the filter or filters are arranged extending from an inlet and/or an outlet part way into the housing.

In a preferred embodiment of the chamber according to the invention, the filter or filters are cylindrically shaped and preferably have their filter openings arranged on the cylindrical surface. This allows a great deal of freedom to vary the filter surface and the number of filter openings.

Alternatively, the filter or filters can be conically shaped with the tip directed towards the interior of the housing. In such cases, the filter openings are advantageously arranged in the conical surface.

With a circular-cylindrical filter the filter openings can be elongated and arranged in the longitudinal direction of the filter. Alternatively, they can be formed as circular arcs around the filter's circular-cylindrical surface. In both cases, a simple and effective shaping is achieved by the use of a relatively simple moulding tool.

If the filter is conically shaped, then the filter openings can be elongated and arranged as circular arcs perpendicular to the generatrix of the cone. Alternatively, the openings can be elongated and arranged along said generatrix. In both cases, and as above, a simple and effective shaping is achieved by the use of a relatively simple moulding tool.

In a preferred embodiment of the chamber according to the present invention, said filter is arranged at an otherwise sealed base of an elongated housing between the interior of the housing and a nipple serving as an inlet and/or outlet. The other end of the housing can hereby be provided with a simple cap with or without inlets and/or outlets. In this way the chamber need comprise just two components.

Particularly if the chamber is intended for blood filtration, it can be advantageous to arrange the filter or filters further in in the housing. This can be achieved by the provision of a non-filtering spacer between the filter and its associated inlet and/or outlet. Thus, when used as a drip chamber, the fall-height is reduced.

A simple design and simple moulding tools are also obtained when the filter or filters are arranged at the one end of an elongated housing which is open at its other end. After moulding, the housing is then sealed at both ends by means of caps which are provided with necessary inlets and/or outlets.

Preferably, the chamber according to the invention, or at least the part which includes the filter, is injection moulded, whereby the filter is obtained by the use of a core which simultaneously creates an inlet to, or an outlet from, the filter.

Said core is suitably conically shaped and may be provided with projections on its surface to create the necessary filter openings.

The ability to increase the variety of filters is made possible when the outer surface of the filter is formed by a core or other mould component which is arranged to be able to be provided with different inserts to obtain desired shaping of the filter.

### BRIEF DESCRIPTION OF THE DRAWINGS:

Figs. 1 to 4 show a first embodiment of the chamber according to the invention.
Figs. 5 and 6 show on a larger scale the filter of the chamber shown in Figs. 1 to 4.
Figs. 7 and 8 show a modified embodiment of the filter.
Fig. 9 illustrates one example of how an injection moulding tool can be formed to produce the filter according to Figs. 5 and 6.
Figs. 10 to 15 show six further embodiments of the chamber according to the invention, whilst
Fig. 16 shows on a larger scale a filter for use in the embodiment according to Fig. 15.

### DETAILED DESCRIPTION OF THE INVENTION:

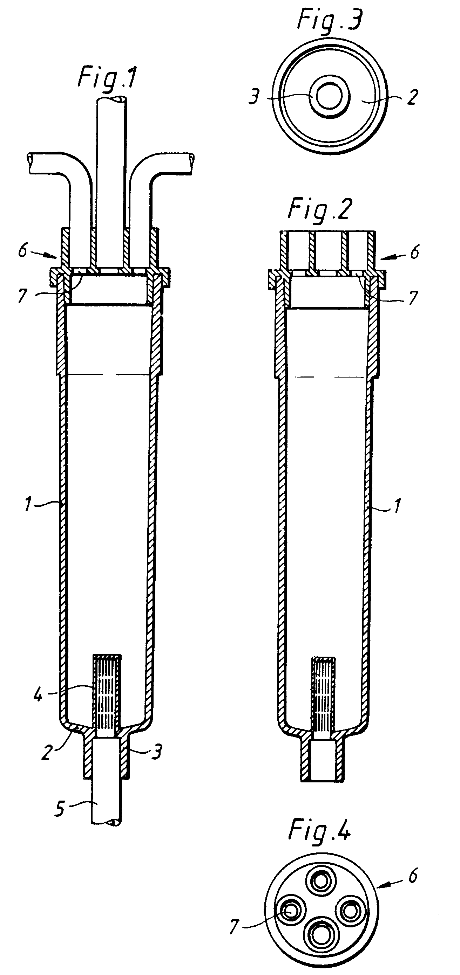

A first embodiment of the invention is shown in Figs. 1 to 6. In this case it consists of an elongated housing 1 with a lower part 2 presenting a nipple 3 and a filter 4. A tube 5 is connected to the nipple 3. The upper part of the chamber is formed by a cap 6 with, in the shown example, four connection openings 7. If the chamber is used in an extracorporeal blood circuit, one of the openings 7 can serve as the inlet for the blood, whilst a second can be connected to a pressure gauge and a third to a level regulator. The fourth opening can be used for sample-withdrawal and/or for the addition of medicine.

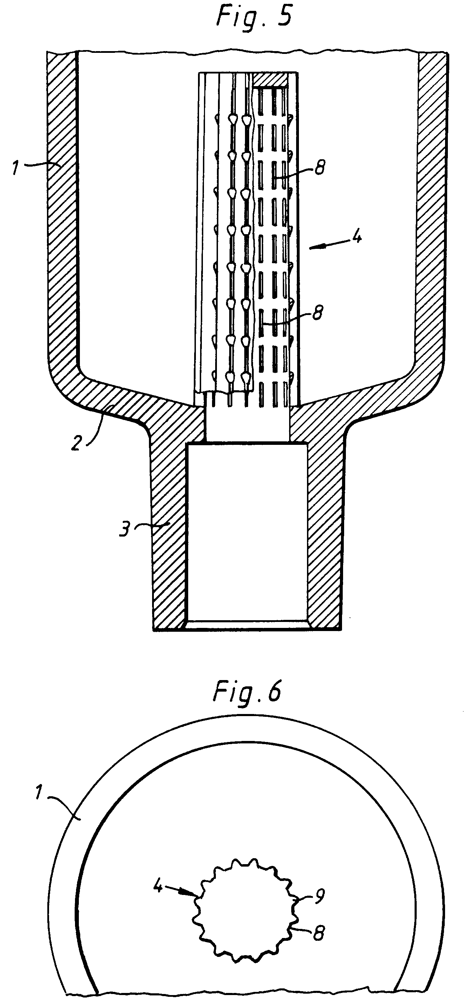

The filter 4 is shown on a larger scale in Figs. 5 and 6. It is thus formed integrally with the housing 1 at its lower part 2. In the shown example, the filter openings 8 are in the form of elongated slots arranged in the longitudinal direction of the housing. The width of the slots should at filtering of blood lie between 0,05 and 0,2 mm and preferably between 0,10 and 0,15 mm. The length of the slots should in the same way lie between 0,5 and 3 mm. Preferably, a length of about 1,5 mm is chosen. The total surface of the slots should be larger than the inner cross section area of the blood inlet tube, preferably at least 50% larger. This in order to reduce the flow resistance of the filter. The filter is strengthened by the provision of reinforcement ribs 9 between the slots 8. (The above mentioned dimensions are valid also for the rest of the embodiments of the filter according to the invention shown in this application.)

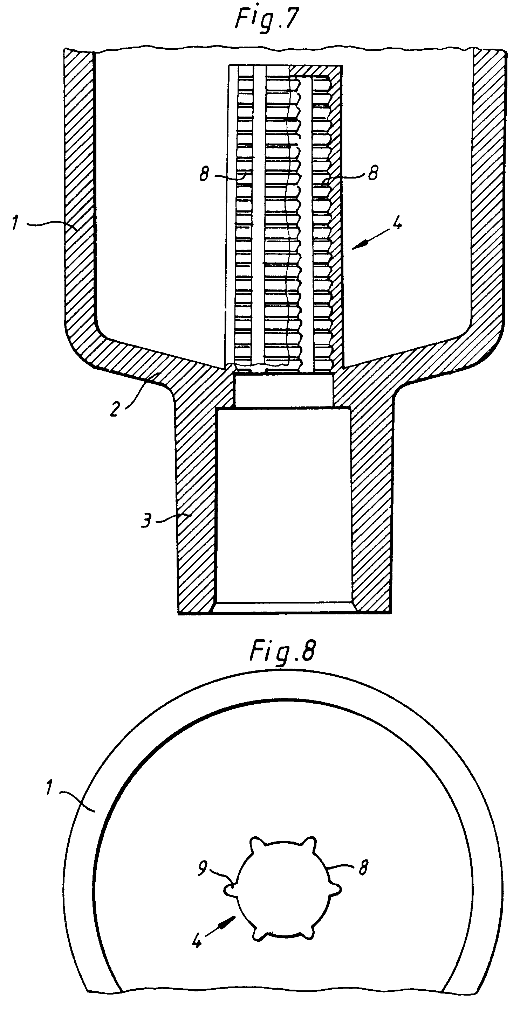

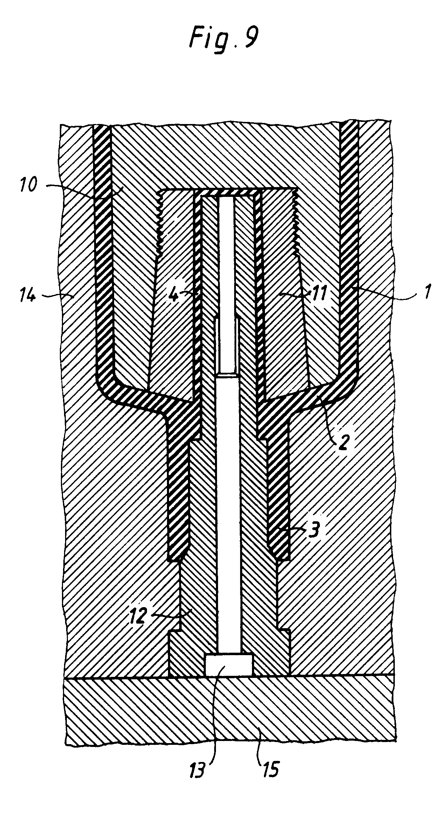

An alternative embodiment of the filter is shown in Figs. 7 and 8. Since this embodiment corresponds essentially with the filter shown in Figs. 5 and 6, the same figure reference numerals have been used. Reference numeral 4 thus denotes the filter, whilst the filter openings are denoted by 8 and a number of longitudinally extending strengthening ribs by 9.

Fig. 9 illustrates an example of how an injection moulding tool can be formed to produce the filters shown in Figs. 1 to 8. The tool consists here of six mould parts which are denoted by 10 to 15. The part 11 can hereby be said to form an insert in the part 10. By exchanging the insert 11, the shape of the filter 4 can be easily altered.

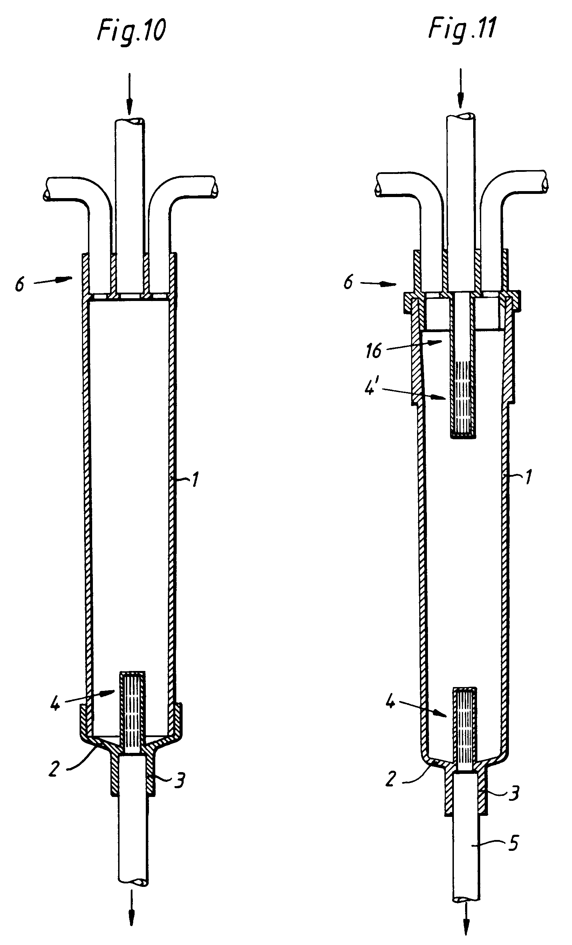

A modified embodiment of the chamber according to the invention is shown in Fig. 10. Since this embodiment corresponds essentially to that described above, the same figure reference numerals have been used. The housing or sleeve is thus denoted by 1 and the lower part by 2. The lower part in this case is formed as a separate injection moulded cap and integrally formed with the filter 4 and the nipple 3. The upper part 6 in this case is integrally formed with the housing 1. Even in this case, the entire chamber consists of just two simple injection moulded components.

A further embodiment of the invention is shown in Fig. 11. The lower part 2 corresponds to that of Fig. 1. The upper part 6 differs from that shown in Fig. 1 in that it is provided with an additional filter 4'. The additional filter occupies a relatively low position within the housing 1 as a result of an extension piece 16.

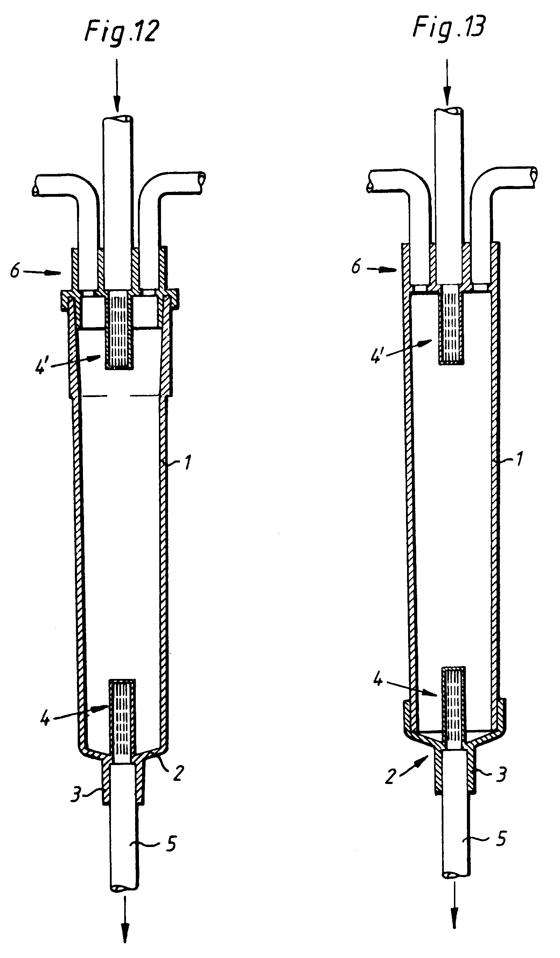

A further embodiment of the invention is shown in Fig. 12. This corresponds essentially to that of Fig. 11, though without the extension piece 16.

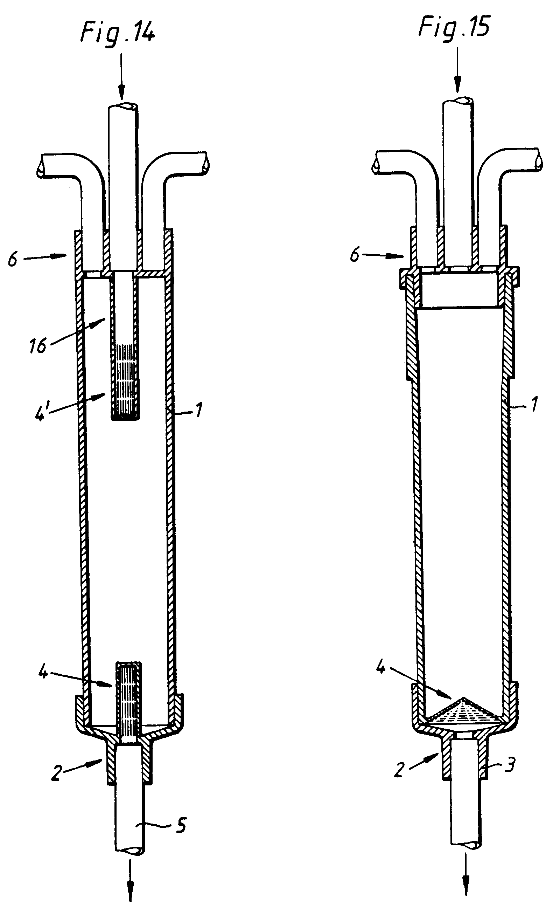

A further embodiment of the invention is shown in Fig. 13. This corresponds essentially to that of Fig. 12, though here the lower part 2 has been replaced by a separate injection moulded cap whilst the upper part 6 is instead integrally formed with the housing 1.

A further embodiment of the invention is shown in Fig. 14. This corresponds essentially to that of Fig. 13, though here, as with the embodiment according to Fig. 11, it is provided with an extension piece 16 to reduce the fall height of blood when the chamber is used as a drip chamber in an extracorporeal blood circuit.

A further embodiment of the invention is shown in Fig. 15. This differs from those described above in that it consists of three parts. This does however offer the advantage that all three parts can be manufactured by means of a very simple injection moulding tool. The lower part 2 can thus be said to comprise the base of the housing 1, the filter 4 integral therewith, as well as an outer cap with the connection nipple 3.

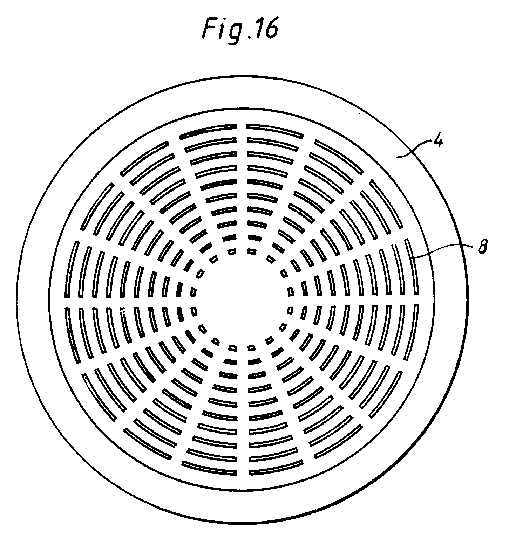

As illustrated in Fig. 16, the conical filter is provided with filter openings in the form of circular arcs arranged perpendicular to the generatrix of the cone. Alternatively, the filter openings can instead be in the form of elongated straight slots arranged in the longitudinal direction of the generatrix of the cone.

The invention is naturally not restricted to only the above described embodiments, but can be varied within the scope of the appended claims. For example it is conceivable that both the shape and size of the filter openings be varied in accordance with circumstances.

## Claims

1. Drip chamber and/or expansion chamber comprising a housing (1) with an upper part (6) and a lower part (2), each of which being provided with one or more inlets and/or outlets (3,7), and at least one filter (4) arranged between said parts, **characterized** in that at least one such filter (4) is integrally formed with one of said parts (2, 6).

2. Chamber according to claim 1, **characterized** in that the filter and its associated part (2, 6) were manufactured simultaneously by injection moulding in a single mould.

3. Chamber according to claim 1, **characterized** in that the housing (1) consists of an elongated, preferably circular-cylindrical sleeve which, at both its ends, is closed by said parts (2, 6), of which one or both can consist of a cap attached to the sleeve.

4. Chamber according to any one of the preceding claims, **characterized** in that the filter or filters (4,4') are arranged extending from an inlet (7) or an outlet (3) part way into the housing (1).

5. Chamber according to any one of the preceding claims, **characterized** in that the filter or filters (4,4') are cylindrically shaped and preferably have their filter openings (8) arranged on the cylindrical surface.

6. Chamber according to claim 1, **characterized** in that the filter or filters (4,4') are conically shaped, preferably with the tip directed towards the interior of the housing (1), with the filter openings (8) being advantageously arranged in the conical surface.

7. Chamber according to any one of claims 3 to 5, **characterized** in that the filter openings (8) are elongated and arranged in the longitudinal direction of the filter (4) and/or are formed as circular arcs around the filter's circular-cylindrical surface.

8. Chamber according to claim 6, **characterized** in that the filter openings (8) are elongated and arranged as circular arcs perpendicular to the generatrix of the cone and/or are straight and arranged along said generatrix.

9. Chamber according to claim 1, **characterized** in that said filter (4) is arranged at an otherwise sealed base (2) of an elongated housing (1) between the interior of the housing and a nipple (3) serving as an inlet and/or outlet (Fig. 1).

10. Chamber according to any one of the preceding claims, **characterized** in that the filter or filters (4,4') are arranged further in in the housing by the provision of a non-filtering spacer (16) between the filter and its associated inlet and/or outlet (Fig. 11).

11. Chamber according to any one of claims 1 to 4, **characterized** in that the filter is arranged at the one end of an elongated housing (1) which is open at its other end, which housing, after moulding, is then sealed at both ends by means of caps (2 and 6) which are provided with necessary inlets and/or outlets (3,7) (Fig. 15).

12. Method of manufacturing a chamber according to any one of the preceding claims, **characterized** in that at least the part which includes the filter (4) is injection moulded, whereby the filter (4) is obtained by the use of a core (13) which simultaneously creates an inlet to, or an outlet (3) from, the filter.

13. Method according to claim 12, **characterized** in that said core (13) is conically shaped and provided with projections on its surface to create required filter openings (8).

14. Method according to claim 12 or 13, **characterized** in that the outer surface of the filter (4) is formed by a core or other mould component (10) which is arranged to be able to be provided with different inserts (11) to obtain desired shaping of the filter.

## Patentansprüche

1. Tropfenkammer und/oder Expansionskammer mit einem Gehäuse (1) mit einem oberen Teil (6) und einem unteren Teil (2), von denen jeder mit einem oder mehreren Einlässen und/oder Auslässen (3, 7) versehen ist, und mit wenigstens einem Filter (4), das zwischen diesen Teilen angeordnet ist, **dadurch gekennzeichnet, daß** wenigstens ein solches Filter (4) einstückig mit einem dieser Teile (2, 6) ausgebildet ist.

2. Kammer nach Anspruch 1, dadurch gekennzeichnet, daß das Filter und das mit ihm verbundene Teil (2, 6) gleichzeitig durch Spritzgießen in einer einzigen Form hergestellt wurden.

3. Kammer nach Anspruch 1, dadurch gekennzeichnet, daß das Gehäuse (1) aus einer länglichen, vorzugsweise kreiszylindrischen Hülse besteht, die an ihren beiden Enden durch die Teile (2, 6) verschlossen ist, von denen eines oder beide aus einer an der Hülse befestigten Kappe bestehen können.

4. Kammer nach einem der vorausgehenden Ansprüche, dadurch gekennzeichnet, daß das Filter oder die Filter (4, 4') so angeordnet sind, daß sie sich von einem Einlaß (7) oder einem Auslaß (3) über einen Teil des Weges in das Gehäuse (1) hinein erstrecken.

5. Kammer nach einem der vorausgehenden Ansprüche, dadurch gekennzeichnet, daß das Filter oder die Filter (4, 4') zylindrisch geformt sind und vorzugsweise ihre Filteröffnungen (8) auf der Zylinderoberfläche angeordnet haben.

6. Kammer nach Anspruch 1, dadurch gekennzeichnet, daß das Filter oder die Filter (4, 4') kegelförmig geformt sind, vorzugsweise mit der Spitze zu dem Inneren des Gehäuses (1) hin gerichtet, wobei die Filteröffnungen (8) günstigerweise in der Kegeloberfläche angeordnet sind.

7. Kammer nach einem der Ansprüche 3 bis 5, dadurch gekennzeichnet, daß die Filteröffnungen (8) länglich und in der Längsrichtung des Filters (4) angeordnet und/oder als Kreisbögen um die kreiszylindrische Oberfläche des Filters herum ausgebildet sind.

8. Kammer nach Anspruch 6, dadurch gekennzeichnet, daß die Filteröffnungen (8) länglich und als Kreisbögen senkrecht zu der Erzeugenden des Kegels angeordnet sind und/oder gerade und entlang dieser Erzeugenden angeordnet sind.

9. Kammer nach Anspruch 1, dadurch gekennzeichnet, daß das Filter (4) an einer im übrigen dicht verschlossenen Basis (2) eines länglichen Gehäuses (1) zwischen dem Inneren des Gehäuses und einem Nippel (3), der als ein Einlaß und/oder Auslaß dient (Figur 1), angeordnet ist.

10. Kammer nach einem der vorausgehenden Ansprüche, dadurch gekennzeichnet, daß das Filter oder die Filter (4, 4') weiter in dem Gehäuse angeordnet sind, indem ein nichtfiltrierender Abstandshalter (16) zwischen dem Filter und dem mit ihm verbundenen Einlaß und/oder Auslaß (Figur 11) vorgesehen ist.

11. Kammer nach einem der Ansprüche 1 bis 4, dadurch gekennzeichnet, daß das Filter an dem einen Ende eines länglichen Gehäuses (1) angeordnet ist, welches an seinem anderen Ende offen ist, wobei dieses Gehäuse nach dem Formen dann an beiden Enden mit Hilfe von Kappen (2 und 6) dicht verschlossen wird, welche mit den erforderlichen Einlässen und/oder Auslässen (3, 7) (Figur 15) versehen sind.

12. Verfahren zur Herstellung einer Kammer nach einem der vorausgehenden Ansprüche, dadurch gekennzeichnet, daß wenigstens der Teil, welcher das Filter (4) einschließt, durch Spritzgießen geformt wird, wobei das Filter (4) durch Verwendung eines Kerns (13) erhalten wird, welcher gleichzeitig einen Einlaß zu dem Filter oder einen Auslaß (3) aus dem Filter erzeugt.

13. Verfahren nach Anspruch 12, dadurch gekennzeichnet, daß der Kern (13) kegelförmig geformt ist und mit Vorsprüngen auf seiner Oberfläche versehen ist, um die erforderlichen Filteröffnungen (8) zu erzeugen.

14. Verfahren nach Anspruch 12 oder 13, dadurch gekennzeichnet, daß die Außenoberfläche des Filters (4) durch einen Kern oder ein anderes Formteil (10) gebildet wird, welches so angeordnet wird, daß es mit unterschiedlichen Einsätzen (11) versehen werden kann, um das erwünschte Formen des Filters zu bekommen.

## Revendications

1. Chambre d'écoulement et/ou chambre d'expansion comprenant un corps (1) constitué d'une partie supérieure (6) et d'une partie inférieure (2), dont chacune comporte une ou plusieurs entrées et/ou sorties (3,7), et au moins un filtre (4) placé entre lesdites parties, caractérisée en ce qu'au moins un tel filtre (4) est formé solidairement avec une des dites parties (2,6).

2. Chambre suivant la revendication 1, caractérisée en ce que le filtre et sa partie associée (2,6) ont été fabriqués simultanément par moulage par injection dans un moule unique.

3. Chambre suivant la revendication 1, caractérisée en ce que le corps (1) est constitué d'un manchon allongé, de préférence cylindrique circulaire, qui est fermé à ses deux extrémités par lesdites parties (2,6) dont l'une ou les deux peuvent consister en un chapeau fixé au manchon.

4. Chambre suivant une quelconque des revendications précédentes, caractérisée en ce que le filtre ou les filtres (4,4') sont disposés de manière à s'étendre,à partir d'une entrée (7) ou d'une sortie (3),dans une partie du corps (1).

5. Chambre suivant une quelconque des revendications précédentes, caractérisée en ce que le filtre ou les filtres (4,4') sont de forme cylindrique et leurs ouvertures de filtration (8) sont de préférence prévues sur la surface cylindrique.

6. Chambre suivant la revendication 1, caractérisée en ce que le filtre ou les filtres (4,4') sont de forme conique, la pointe étant de préférence dirigée vers l'intérieur du corps (1), les ouvertures de filtration (8) étant avantageusement prévues dans la surface conique.

7. Chambre suivant une quelconque des revendications 3 à 5, caractérisée en ce que les ouvertures de filtration (8) sont allongées et disposées dans la direction longitudinale du filtre (4) et/ou elles sont sous la forme d'arcs de cercle autour de la surface cylindrique circulaire du filtre.

8. Chambre suivant la revendication 6, caractérisée en ce que les ouvertures de filtration (8) sont allongées et disposées en arcs de cercle perpendiculaires à la génératrice du cône et/ou elles sont rectilignes et disposées le long de la dite génératrice.

9. Chambre suivant la revendication 1, caractérisée en ce que ledit filtre (4) est placé à l'endroit d'une base d'autre part fermée (2) d'un corps allongé (1), entre l'intérieur du corps et un raccord (3) servant d'entrée et/ou de sortie (fig. 1).

10. Chambre suivant une quelconque des revendications précédentes, caractérisée en ce que le filtre ou les filtres (4,4') sont disposés plus loin à l'intérieur du corps par interposition d'un élément d'espacement non filtrant (16) entre le filtre et son entrée et/ou sortie associée (fig. 11).

11. Chambre suivant une quelconque des revendications 1 à 4, caractérisée en ce que le filtre est placé à la première extrémité d'un corps allongé (1) qui est ouvert a son autre extrémité, ce corps, après moulage, étant ensuite fermé aux deux extrémités au moyen de chapeaux (2 et 6) qui sont pourvus des entrées et/ou sorties nécessaires (3,7) (fig. 15).

12. Procédé de fabrication d'une chambre suivant une quelconque des revendications précédentes, caractérisé en ce qu'au moins la partie qui comprend le filtre (4) est moulée par injection, de sorte que le filtre (4) est obtenu par l'utilisation d'un noyau (13) qui engendre simultanément une entrée au filtre ou une sortie (3) du filtre.

13. Procédé suivant la revendication 12, caractérisé en ce que ledit noyau (13) est de forme conique et comporte des protubérances sur sa surface pour engendrer les ouvertures de filtration requises (8).

14. Procédé suivant la revendication 12 ou 13, caractérisé en ce que la surface extérieure du filtre (4) est formée par un noyau ou un autre composant du moule (10) qui est agencé de manière à pouvoir recevoir différents inserts (11) afin d'obtenir la configuration désirée du filtre.
